# EUROPEAN PATENT APPLICATION

(11) **EP 3 858 360 A1**
(43) Date of publication of application: **04.08.2021**
(21) Application number: 20305076.0
(22) Date of filing: 29.01.2020
(51) Int. Cl.: A61K 31/5377, A61K 9/00, A61K 31/675, A61P 25/02

(54) **APREPITANT FOR USE IN THE TOPICAL TREATMENT OF FACIAL NEURALGIA**

(71) Applicant: ASSISTANCE PUBLIQUE, HOPITAUX DE PARIS, 75004 Paris (FR)
(72) Inventor: GRECO, Céline, 75012 PARIS (FR)
(74) Representative: Gevers & Orès

(57) **Abstract**

The present invention relates to a compound selected from aprepitant, prodrugs thereof and pharmaceutically acceptable salts thereof, or a composition comprising such a compound, for use in the topical treatment and prevention of facial neuralgia, in particular trigeminal neuralgia.

## Description

The present invention relates to a compound selected from aprepitant, prodrugs thereof and pharmaceutically acceptable salts thereof, or a composition comprising such a compound, for use in the topical treatment and prevention of facial neuralgia, in particular trigeminal neuralgia.

Trigeminal neuralgia is a severe paroxysmal, lancinating facial pain due to a disorder of the trigeminal nerve ("fifth cranial nerve"). It is usually classified into two main types: typical and atypical trigeminal neuralgia. The typical form results in episodes of severe, sudden, shock-like pain in one side of the face that lasts for seconds to a few minutes. The atypical form results in a constant burning pain that is less severe. Both forms may occur in the same patient. Episodes may be triggered by anything touching the face or teeth, including shaving, applying makeup, brushing teeth, eating, drinking or talking - or even a light breeze. Trigerminal neuralgia is one of the most painful conditions, and can sometimes result in depression. It usually begins in people over 50 years old, but it can occur at any age. It is estimated that 5 to 30 cases in 100,000 people per year develop this condition.

Generally, pain is relieved using medication. The administration of anticonvulsants, such as carbamazepine or oxcarbazepine, is usually the initial treatment but it is not effective for all patients and often associated with side effects. Some opioids can also be used but they are not usually effective in the typical form. When no improvement is obtained or when a patient suffers serious side effects of the medications, surgery may also be tried.

There thus remains a genuine need for an alternative and effective treatment having no or a less side effects compared with actual treatments.

The present invention is believed to meet such need by providing a topical composition for treating or preventing facial neuralgia.

Aprepitant of formula (I) belongs to the class of substance P antagonists.

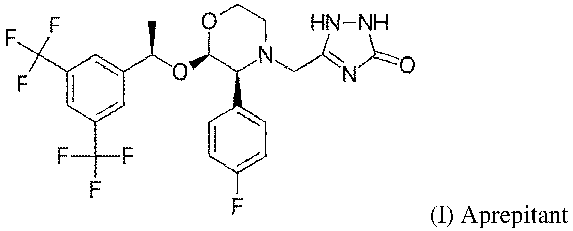

Aprepitant is generally used as an anti-emetic drug for treating chemotherapy-induced nausea and vomiting. Aprepitant acts on the Neurokinin 1 (NK1) receptor which has key regulating functions in the central and peripheral nervous systems. The dominating natural ligand of the NK1 receptors is substance P, a neuropeptide from the family of tachykinins. Substance P is abundantly and widely distributed in the mammalian central nervous system and other tissues and is able to induce emesis by binding to NK1 receptors located in the regions of the brain involved in the regulation of emesis.

The Inventors have in a surprising manner demonstrated the efficacy of aprepitant in the topical treatment and prevention of facial neuralgia, in particular of trigeminal neuralgia, with no side effects compared with actual treatments.

Thus, the present invention concerns a compound selected from aprepitant, prodrugs thereof and pharmaceutically acceptable salts thereof, for use in the topical treatment or prevention of a facial neuralgia, in particular of trigeminal neuralgia.

The present invention further relates to a topical composition comprising a compound according to the invention as active ingredient and at least one pharmaceutically acceptable excipient, for use in the treatment or prevention of facial neuralgia, in particular trigeminal neuralgia.

The present invention further relates to a method for treating and/or preventing facial neuralgia, in particular trigeminal neuralgia, by means of topical administration, to a patient in need thereof, of an effective amount of a compound or composition according to the invention.

The present invention further relates to the use of a compound or composition comprising a compound according to the invention for the manufacture of a topical medication for treating and/or preventing facial neuralgia, in particular trigeminal neuralgia.

"Aprepitant" as used herein (also known via its brand name Emend®) refers to the molecule 3-[[(2R,3S)-2-[(1R)-1-[3,5-Bis(trifluoromethyl)phenyl]ethoxy]-3-(4-fluoro phenyl)morpholino]methyl]-1H-1,2,4-triazol-5(4H)-one, enantiomers, racemic mixtures, polymorphs, salts, solvates, esters or hydrates thereof.

In the present invention, "prodrug" designates a compound that, after administration, is metabolized (*i.e.* converted within the body) into a pharmacologically active drug. Prodrugs of aprepitant include, but are not limited to, fosaprepitant.

"Fosaprepitant" as used herein (also known via its brand name Ivemend®) refers to the molecule of formula (II), enantiomers, racemic mixtures, polymorphs, salts, solvates, esters or hydrates thereof.

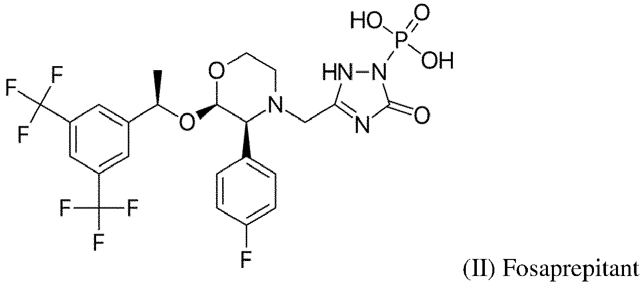

Other NK1 antagonists differing from aprepitant or from fosaprepitant by minor chemical modifications, such as rolapitant, casopitant, netupitant, and maropitant, can also be used according to the invention.

In the present invention, "pharmaceutically acceptable" is intended to mean that which is useful in the preparation of a pharmaceutical composition, generally safe, nontoxic and neither biologically nor otherwise undesirable and acceptable for both veterinary and human pharmaceutical use.

"Pharmaceutically acceptable salt" of a compound is intended to mean a salt that is pharmaceutically acceptable, as defined herein, and that has the desired pharmacological activity of the parent compound.

Salts of aprepitant include salts of acidic or basic groups present in compounds of the application. Pharmaceutically acceptable acid addition salts include, but are not limited to, hydrochloride, hydrobromide, hydroiodide, nitrate, sulfate, bisulfate, phosphate, acid phosphate, isonicotinate, acetate, lactate, salicylate, citrate, tartrate, pantothenate, bitartrate, ascorbate, succinate, maleate, gentisinate, fumarate, gluconate, glucaronate, saccharate, formate, benzoate, glutamate, or pamoate salts. Certain compounds of the invention can form pharmaceutically acceptable salts with various amino acids. Suitable base salts include, but are not limited to, aluminum, calcium, lithium, magnesium, potassium, sodium, zinc, or diethanolamine salts. Potassium salts include potassium chloride, potassium bicarbonate, potassium phosphate, gluconate, potassium citrate, or the like.

In the present invention, the term "neuralgia" designates a severe, shooting pain that occurs due to a damaged or irritated nerve.

"Facial neuralgia" generally refers to a neuralgia located in the face of a subject. In particular, the expression "facial neuralgia" often designates the "trigeminal neuralgia" which is a specific kind of facial pain that involves the trigeminal nerve (or fifth cranial nerve). The pain typically involves the lower face and jaw, although sometimes it affects the area around the nose and above the eye. This intense, stabbing, electric shock-like pain is caused by irritation of the trigeminal nerve, which sends branches to the forehead, cheek and lower jaw. It is usually limited to one side of the face.

By "treatment" is meant, according to the present invention, the inhibition of the development of, more particularly the regression of, preferably the disappearance of the facial pain.

By "prevention" is meant, according to the present invention, to prevent or delay the appearance of the facial pain.

The treatment or prevention according to the invention applies to humans or animals.

By "topical" is meant, according to the present invention, that the compound or composition according to the invention will be administered by application on the skin surface or the mucous membranes.

The topical composition according to the invention may in particular be in any form allowing topical application, such as a cream, a gel, an ointment, a solution, a lotion, a spray, an aerosol spray, an aerosol foam or a patch. Preferably, the composition according to the invention will be in cream or gel form.

In an embodiment, the topical composition of the invention can be applied to the skin by an applicator, such as a roll-on, a stick, an impregnated wipe or an impregnated glove.

In an embodiment, the composition of the invention can also be dispensed from a pump pack or from an aerosol container.

In an embodiment, the topical composition of the invention comprises a compound according to the invention in a concentration from 0.1 to 30%, in particular from 0.5% to 20%, more particularly from 1% to 10%, by weight relative to the weight of the final composition.

In an embodiment, the topical composition of the invention comprises a compound according to the invention in a concentration of 0.1%, 0.2%, 0.3%, 0.4%, 0.5%, 0.6%, 0.7%, 0.8%, 0.9%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% by weight relative to the weight of the final composition.

In an embodiment, the topical composition of the invention is formulated into unit dose form.

The compound or topical composition according to the invention will be administered one or more times per day, the duration being variable according to the pain intensity and easily adjustable by the person skilled in the art or the practitioner.

In an embodiment, the topical composition of the invention is applied to the affected area once daily, twice daily, three times daily, once every second day, three times weekly, twice weekly or once weekly.

In an embodiment, the topical composition of the invention is applied before a pain episode.

In an embodiment, the topical composition of the invention is applied during a pain episode.

By "pharmaceutically acceptable excipient" is meant, according to the invention, an excipient that is compatible with the other ingredients of the composition and that produces no adverse effect, allergic reaction or other undesirable reaction when it is administered to a human or an animal.

According to the invention, by "excipient" is meant in particular one or more surfactants, for example macrogols, hydroxy stearates, ethoxylated fatty acid esters, ethoxylated fatty alcohols; one or more solvents, such as for example octyldodecanol, propylene glycol dicaprylocaprate; one or more hydrosoluble polymers, for example PVP, hyaluronic acid or sodium hyaluronate; one or more thickeners such as for example natural or semisynthetic gums; one or more gelling agents, for example carbomers; one or more inorganic fillers, for example zinc oxide, talc, clays; one or more emulsifiers such as cetearyl alcohol; one or more preservatives, for example phenoxyethanol; one or more antibacterials; one or more antiseptics; one or more antioxidants, for example tocopherol acetate; one or more chelating agents, for example EDTA; one or more pigments; one or more fragrances; one or more colorants; one or more pH adjustors such as salts, acids, bases; or a mixture thereof.

In an embodiment, the topical composition of the invention further comprises a penetration enhancer.

By "penetration enhancer" is meant, according to the invention, a chemical substance that promotes the transdermal penetration of the drug applied at the surface of the skin. For example, a penetration enhancer can be selected among alcohols, amides, esters, glycols, fatty acids, pyrrolidones, sulfoxides, surfactants, terpenes, urea, cyclodextrins, water, vitamin E or phospholipids.

In an embodiment, the topical composition further comprises a base allowing transdermal diffusion of said compound. In particular, such a base may be selected from the group comprising Excipial Hydrocrème®, Codexial Obase®, Pentravan®, Pentravan® Plus, Phytobase®, Lipovan® and Pluronic Lecithin Organogel (PLO), preferably Pentravan®, preferably Excipial Hydrocrème® or Codexial Obase®.

In an embodiment, the topical composition of the invention does not contain any anticonvulsant compound.

In an embodiment, the topical composition of the invention comprises aprepitant in a concentration from 0.5 to 20%, preferably in a concentration of 1.5% by weight relative to the weight of the final composition..

In an embodiment, the topical composition of the invention comprises aprepitant in a concentration from 0.5 to 20% by weight relative to the weight of the final composition in a base of Excipial Hydrocrème®.

The following Examples are put forth so as to provide those of ordinary skill in the art with a complete disclosure and description of how to make and use the present invention, and are not intended to limit the scope of what the Inventors regard as their invention nor are they intended to represent that the experiments below are all or the only experiments performed. While the present invention has been described with reference to the specific embodiments thereof, it should be understood by those skilled in the art that various changes may be made and equivalents may be substituted without departing from the true spirit and scope of the invention. In addition, many modifications may be made to adapt a particular situation, material, composition of matter, process, process step or steps, to the objective, spirit and scope of the present invention. All such modifications are intended to be within the scope of the claims appended hereto.

### EXAMPLES

### Example 1

Three patients suffering from facial neuralgia were treated with success with topical applications of aprepitant cream.
- Patient 1, a man aged 66, suffered from trigeminal neuralgia since 30 years.
   No relieved by: ketamine, multi anti-depressants and anti-epileptics, opioids.
   The patient took 8 actiskenan per os every day. He tried aprepitan cream at 0.5% with moderate beneficial effects then tried aprepitan cream at 1.5% with a very good relief of pain in one month. He was able to stop completely opioids.
- Patient 2, a woman aged 41, suffered from facial neuralgia since 1 year.
   No relieved by: pregabalin, gabapentin, methadone, duloxetine and amitriptyline.
   She tried aprepitan cream at 1.5% with a very good relief of pain in one month. She was able to stop pregabalin and halve the daily methadone doses.
- Patient 3, a man aged 68, suffered from post-zooster facial neuralgia since 2013.
   No relieved by: amitriptylin, tramadol, pregabalin, acupuncture and Versatis patch.
   The patient took pregabalin 300mg twice a day and tramadol 50mg every 6h. He tried aprepitan cream at 1.5% with a very good relief of pain in one month and was able to stop pregabalin and to decrease tramadol.

## Claims

1. A compound selected from aprepitant, prodrugs thereof and pharmaceutically acceptable salts thereof, for use in the topical treatment or prevention of facial neuralgia, in particular trigeminal neuralgia.

2. A compound for use according to claim 1, said compound being aprepitant.

3. A compound for use according to claim 1, said compound being fosaprepitant.

4. A topical composition comprising a compound selected from aprepitant, prodrugs thereof and pharmaceutically acceptable salts thereof, for use in the treatment or prevention of facial neuralgia, in particular trigeminal neuralgia.

5. The topical composition for use according to claim 4, wherein said compound is aprepitant.

6. The topical composition for use according to claim 4, wherein said compound is fosaprepitant.

7. The topical composition for use according to any one of claims 4-6, wherein the composition is in the form of a solution, a gel, a cream, an ointment, a lotion, a spray, an aerosol spray, an aerosol foam or a patch.

8. The topical composition for use according to any one of claims 4-7, wherein the compound is in a concentration from 0.1% to 30%, preferably in a concentration of 2%, by weight relative to the weight of the final composition.

9. The topical composition for use according to any one of claims 4-8, wherein the topical composition is formulated into unit dose form.

10. The topical composition for use according to any one of claims 4-9, wherein the topical composition is applied to the affected area once daily, twice daily, three times daily, once every second day, three times weekly, twice weekly or once weekly.

11. The topical composition for use according to any one of claims 4-10, further comprising a penetration enhancer.
